# EUROPÄISCHE PATENTSCHRIFT

(11) **EP 1 840 235 B1**
(45) Veröffentlichungstag und Bekanntmachung des Hinweises auf die Patenterteilung: **18.12.2013**
(21) Anmeldenummer: 07005877.1
(22) Anmeldetag: 22.03.2007
(51) Int. Cl.: C22C 23/04, C22C 23/00

(54) **Magnesiumlegierung und dazugehöriges Herstellungsverfahren**
Magnesium alloy and corresponding production method
Alliage de magnésium et son procédé de fabrication

(30) Priorität: 31.03.2006 DE 102006015457
(43) Veröffentlichungstag der Anmeldung: 03.10.2007
(73) Patentinhaber: Biotronik VI Patent AG, 6341 Baar (CH)
(72) Erfinder: Gerold, Bodo, Dr., 91225 Zellingen (DE); Hänzi, Anja, 5400 Baden (CH); Löffler, Jörg, Prof. Dr., 8049 Zürich (CH); Müller, Heinz, Dr., 91052 Erlangen (DE); Uggowitzer, Peter, Prof. Dr., 8913 Ottenbach (CH)
(74) Vertreter: Lindner-Vogt, Karin L.

(56) Entgegenhaltungen:
- EP-A1- 0 531 165
- EP-A2- 0 407 964
- JP-A- 8 134 581
- JP-A- 2004 099 941

## Beschreibung

Die Erfindung betrifft eine neue Magnesiumlegierung und seine Verwendung sowie ein dazugehöriges Herstellungsverfahren für die Magnesiumlegierung.

### Technologischer Hintergrund und Stand der Technik

Bekannt sind zahlreiche Magnesiumlegierungen unterschiedlichster Zusammensetzung und Verwendbarkeit. Vorliegend wird unter dem Begriff Magnesiumlegierung eine Gruppe von Legierungen verstanden, die neben Magnesium als Hauptbestandteil Zusätze (gewöhnlich bis ca. 10%) an Aluminium, Mangan, Zink, Kupfer, Nickel, Cer-Mischmetall und anderen Seltenerdmetallen, Silber, Zirconium, Silizium usw. enthalten. Magnesiumlegierungen werden eingeteilt in Mg-Knetlegierungen (Basis üblicherweise Mg-Mn, Mg-Al-Zn) und Mg-Gusslegierungen; letztere werden ihrerseits in Sandguss, Kokillenguss und Druckguss oder nach Legierungsbestandteilen untergliedert. Magnesiumlegierungen können nach den meisten bekannten metallurgischen Urform- und Umformverfahren verarbeitet werden.

Die Legierungszusätze bestimmen maßgeblich die Eigenschaften des metallischen Werkstoffs. Bekannt ist beispielsweise, dass Aluminium-Gehalte über ca. 10 Gew.% zum Verspröden der Legierungen führen. Zink und besonders Zirconium erhöhen die Zähigkeit, während Mangan die Korrosionsbeständigkeit verbessert. Beryllium-Zusätze von einigen ppm verringern merklich die Oxidationsneigung des schmelzflüssigen Metalls, sind jedoch aufgrund ihrer Toxizität unerwünscht. Seltenerdmetalle und Thorium steigern die Warmfestigkeit. Der Schmelzpunkt der Legierungen liegt üblicherweise zwischen 590° und 650°C.

Haupteinsatzgebiete von Magnesiumlegierungen sind Luftfahrt, Maschinenbau aller Art, optische Geräte, Elektrotechnik, Elektronik, Transportmittel, Büromaschinen und Haushaltsmaschinen, und zwar generell in Bereichen, bei denen es auf Festigkeit und Steifigkeit bei möglichst geringem Gewicht ankommt sowie niedrige Fertigungskosten bei großen Serien gefordert werden. Zunehmende Bedeutung erhalten Magnesiumlegierungen im Motorenbau für Kraftfahrzeuge. Eine Spezialanwendung betrifft die Verwendung von biodegradierbaren Magnesiumlegierungen in der Medizintechnik, insbesondere für vaskuläre und orthopädische Implantate.

Eine Limitierung bekannter Magnesiumlegierungen besteht insbesondere in der für bestimmte Verarbeitungsverfahren und Einsatzzwecke unzureichenden Duktilität des Werkstoffs. Ein Ansatzpunkt zur Verbesserung könnte in der Verminderung der Korngröße des metallischen Gefüges liegen (Feinen). Das Feinen umfasst alle metallurgischen Maßnahmen, die zu einer kleinen Korngröße einer Legierung führen. Allgemein setzt dies die Erhöhung der Keimzahl in der Schmelze beim Erstarren oder im Festkörper durch feindisperse Ausscheidungen voraus. Feinen wirkt sich vorteilhaft auf die mechanischen Eigenschaften, insbesondere die Duktilität der Legierung aus. Im Bereich der Magnesiumlegierungen wurden sehr geringe Korngrößen bisher nur im kleinpräparativem Maßstab erzielt, etwa durch ECAP/ECAE-Verfahren (ECAP: equal channel angular pressing; ECAE: equal channel angular extrusion). Die genannten Verfahren lassen sich jedoch nicht großtechnisch umsetzen; bisher wurden nur kleine Volumina (wenige cm³) hochfeinkörniger Legierungen vorwiegend mit der technischen Magnesiumlegierung AZ31 erzeugt. Erkenntnisse allgemeingültiger Natur über die für das Feinen notwendigen Legierungskomponenten oder gar deren Anteil in Magnesiumlegierungen liegen bisher nur in unzureichender Form vor.

Des Weiteren ist aus Dokument EP 0 407 964 A2 eine feinkörnige hochfeste Legierung auf Magnesium-Basis der Zusammensetzungen MgₐX_{b}, MgₐX_{c}M_{d}, MgₐX_{c}Lnₑ, oder MgₐX_{c}M_{d}Lnₑ bekannt, wobei X ausgewählt ist einem oder mehreren Elementen aus der Gruppe Cu, Ni, Sn und Zn; M ausgewählt ist einem oder mehreren Elementen aus der Gruppe Al, Si und Ca; Ln ausgewählt ist einem oder mehreren Elementen aus der Gruppe Y, La, Ce, Nd, Sm oder ein Mischmetall; und a, c, d und e Atomprozentsätze sind, die innerhalb der folgenden Bereiche fallen: 40≤a≤95, 1≤c≤35, 1≤d≤25 und 3≤e≤25.

Es besteht daher ein anhaltender Bedarf an Magnesiumlegierungen, die ein Feinen auch unter Rückgriff herkömmlicher großtechnischer Verfahren erlauben. Weiterhin besteht Bedarf an einer Magnesiumlegierung, deren Korngröße mit Hinsicht auf eine verbesserte Duktilität gegenüber herkömmlichen Legierungen verringert ist. Ferner besteht Bedarf an einem technisch im größeren Maßstab umsetzbaren Darstellungsverfahren für eine feinkörnige Magnesiumlegierung. Schließlich ist es mit Hinsicht auf ökologische Aspekte als auch eine medizintechnische Verwendung der Legierungen notwendig die Legierungskomponenten unter toxikologischen bzw. biokompatiblen Gesichtspunkten auszuwählen; letzteres insbesondere unter Vermeidung des in vielen Magnesiumlegierungen präsenten Aluminiums.

### Erfindungsgemäße Lösung

Ein erster Aspekt der Erfindung betrifft eine neue Magnesiumlegierung der Zusammensetzung

| | |
|---|---|
| Y: | 0,5 - 10 |
| Zn: | 0,5 - 6 |
| Ca: | 0,05 - 1 |
| Mn: | 0 - 0,5 |
| Ag: | 0 - 1 |
| Ce: | 0 - 1 |
| Zr: | 0 - 1 oder Si: 0 - 0,4 |

wobei sich die Angaben auf Gew.% an der Legierung beziehen und Mg sowie herstellungsbedingte Verunreinigungen den auf 100 Gew.% verbleibenden Restanteil an der Legierung einnehmen. Die Legierung zeichnet sich dadurch aus, dass sie - bei geeigneter Behandlung - in ein sehr feinkörniges Gefüge (insbesondere mit Korngrößen < 20 µm) überführbar ist. Ferner sind die Legierungskomponenten toxikologisch nicht oder kaum relevant, so dass insbesondere eine medizintechnische Anwendung prädestiniert erscheint. Vorzugsweise liegt der Anteil von Mn im Bereich von 0,05 bis 0,5 Gew.%.

Der Erfindung liegt unter anderem die Erkenntnis zu Grunde, dass die genannten Legierungselemente mit den genannten Anteilen einerseits beim Erstarren aus der Schmelze durch ihre Anreicherung an der Erstarrungsfront und andererseits bei der Warmumformung durch die Bildung intermetallischer Phasen das Kornwachstum hemmen. Bei grobkörniger Gefügestruktur wird die plastische Verformung dominiert durch Versetzungsbewegungen auf der Basalebene und durch Zwillingsbildung; feinkörnige Magnesiumgefüge erlauben dagegen schon bei Raumtemperatur die Aktivierung von Prismen- und Pyramidalgleitung, was die Duktilität des Werkstoffs wesentlich verbessert. Die während der Erstarrung an der Erstarrungsfront angereicherten Legierungselemente mit hohem Q-Faktor (grain growth restriction factor) verzögern das Kornwachstum und tragen somit bei zu einer geringen Korngröße des Gussgefüges. Die in der erstarrten Magnesiumlegierung vorliegenden sehr kleinen intermetallischen Phasen der weiter unten noch näher erläuterten Zusammensetzung beeinflussen die Rekristallisation bei der Warmumformung des Gussgefüges und tragen ebenfalls wesentlich zur feinen Gefügeausbildung bei. Die Gegenwart der intermetallischen Phasen verbessert dabei nicht nur die Duktilität des Werkstoffs, sondern beeinflusst auch maßgeblich dessen Korrosionseigenschaften. Letzteres hat insbesondere Bedeutung, wenn die Magnesiumlegierung zur Herstellung biodegradierbarer Implantate (bevorzugt Stents) eingesetzt wird. In der Regel wird die Gegenwart feiner intermetallischer Phasen (anstelle grobkörniger Phasen) mit spezieller Stöchiometrie den Korrosionswiderstand weniger reduzieren. Ziel ist es, ein annähernd einphasiges Gefüge herzustellen und grob mehrphasige Gefüge zu vermeiden.

Bevorzugte Magnesiumlegierungen ergeben sich durch die nachfolgenden Beschränkungen der Anteile des angegebenen Legierungsbestandteils oder mehrerer dieser Legierungsbestandteile (Angaben in Gew.%):

| | |
|---|---|
| Y: | 0,5 - 4; |
| Zn: | 0,5 - 3,0; insbesondere 0,8 - 2,5; |
| Ca: | 0,05 - 0,3; insbesondere 0,05 - 0,2; |
| Mn: | 0 - 0,25; insbesondere 0,05 - 0,25; |
| Ag: | 0,05 - 0,6; |
| Ce: | 0 - 0,5; |
| Zr: | 0 - 0,7; insbesondere 0,3 - 0,7 oder Si:0 - 0,25; insbesondere 0,05 - 0,25. |

Die genannten Legierungszusammensetzungen scheinen ersten Vorversuchen und theoretischen Überlegungen zu Folge besonders geeignet hoch-feinkörnige Werkstoffe auf Magnesiumbasis zu liefern (insbesondere mit Korngrößen < 20 µm). Zu beachten ist, dass ein Nebeneinander von Zirconium und Silizium zu vermeiden ist, da sich für die Zwecke des Feinens ungeeignete intermetallische Phasen aus den beiden Elementen bilden.

Eine Legierung im erfindungsgemäßen Sinne bezieht sich auf einen metallischen ein- oder mehrphasigen Werkstoff eines Zwei- oder Mehrstoffsystems, dessen Ausgangskomponenten (Legierungselemente) metallurgisch miteinander in Wechselwirkung treten und dabei zur Bildung neuer Phasen führen (Mischkristalle, intermetallische Verbindungen, Überstrukturen). Die Magnesiumlegierung enthält als Hauptbestandteil Magnesium.

Bevorzugt ist - insbesondere auch im Zusammenspiel mit den vorgenannten bevorzugten Zusammensetzungsvarianten der Magnesiumlegierung -, dass die Magnesiumlegierung ein oder mehrere intermetallische Phasen bestehend aus
(i) Mg und einem oder mehreren Elementen ausgewählt aus der Gruppe: Zn, Ca, Mn, Ag, Ce, Zr, Si und Y; oder
(ii) 2 oder mehr Elementen ausgewählt aus der Gruppe: Zn, Ca, Mn, Ag, Ce, Zr, Si und Y
enthält. Das Vorhandensein einer oder mehrerer intermetallischer Phasen der genannten Zusammensetzungen ist ein wesentliches Indiz für die Eignung der Legierung zur Herstellung eines feinkörnigen Werkstoffs (mit vorzugsweise einer Korngröße < 20 µm), sofern diese Legierung nicht bereits als feinkörniger Werkstoff vorliegt. Besonders bevorzugt sind ein oder mehrere intermetallische Phasen ausgewählt aus der Gruppe: Ca₂M₉₆Zn₃, AgMg₄, Mn₂Zr, ZnZr, Zn₂Zr, MgZn, MgZn₂, Mg₂Si, Mg₃Y₂Zn₃, Mg₃YZn₆, Mg₁₂YZₙ und M9₂₄Y₅ vorhanden.

Intermetallische Phasen (Verbindungen) sind im Gefüge von Legierungen vorhandene chemische Verbindungen aus zwei oder mehr metallischen Elementen, deren Struktur sich von jener der sie bildenden Metalle deutlich unterscheidet. Neben kubischen treten auch tetragonale und komplexere Strukturen auf. Im Gitter liegen außer metallischen auch Atom- und Ionenbindungsanteile vor. Neben intermetallischen Phasen mit stöchiometrischer Zusammensetzung entsprechend den vorhandenen Valenzen gibt es solche, bei denen diese exakte Zusammensetzung nur einen Sonderfall in einem breiten Homogenitätsbereich darstellt. Dies folgt aus dem Bestreben der beteiligten Metalle, bei den gegebenen Bindungsverhältnissen ein Gitter mit möglichst hoher Koordinationszahl und Packungsdichte auszubilden. Metallbindung und -eigenschaften sind dabei umso stärker ausgeprägt, je höher die Koordinationszahl ist, z.B. bei der Gruppe der Laves-Phasen. Hume-Rothery-Phasen bilden breite Homogenitätsbereiche, ebenso intermetallische Phasen mit Einlagerungsstrukturen. Bei Anwachsen der homöopolaren bzw. heteropolaren Bindungsanteile kristallisieren die intermetallischen Phasen in Gittern mit niederer Koordinationszahl, wie z.B. die Zintl-Phasen. Die Schmelzpunkte der intermetallischen Phasen liegen deutlich über denen der Metallkomponenten, ihre elektrische Leitfähigkeit ist erheblich niedriger. In metallischen Gefügen als Phasen auftretende intermetallische Verbindungen können in feindisperser Form die Festigkeit steigern oder in gröberer Form zu einer Versprödung der Legierung führen, darüber hinaus können sie die Korrosionsbeständigkeit beeinträchtigen.

Bevorzugt ist - insbesondere im Zusammenhang mit den vorherig beschriebenen Ausführungsvarianten - ein Volumenanteil der intermetallischen Phasen an der Magnesiumlegierung < 3 Vol.%, insbesondere < 2 Vol.%, besonders bevorzugt < 1 Vol.%. Alternativ oder ergänzend hierzu ist vorzugsweise:
(i) eine Korngröße der intermetallischen Phasen < 3 µm, insbesondere < 1 µm; und/oder
(ii) eine Korngröße der Legierung < 20 µm, insbesondere < 10 µm.

Unter dem Begriff "Korngröße" wird vorliegend der Durchschnittswert des Durchmessers der in einem metallographischen Schliffbild vorliegenden Kristallite verstanden. Magnesiumlegierungen der vorgenannten Ausführungsformen weisen für die Verarbeitung und ihre spätere Zweckbestimmung besonders günstige Eigenschaften gegenüber herkömmlichen Magnesiumlegierungen auf: ihre Duktilität ist deutlich erhöht. Unter Duktilität (oder auch Zähigkeit, Verformungsvermögen) wird allgemein die Fähigkeit eines metallischen Werkstoffes verstanden, sich unter gegebenen Bedingungen bei hinreichend hohen mechanischen Beanspruchungen bleibend zu verformen, bevor Rissbildung eintritt. Diese Fähigkeit ist für viele Bauteile von großer Bedeutung, da örtlich auftretende mechanische Spannungshöchstwerte nur von einem duktilen Werkstoff rissfrei durch bleibende Verformung unter gleichzeitiger Kaltverfestigung abgebaut werden können. Gerade dieser Aspekt macht den Einsatz der erfindungsgemäßen Magnesiumlegierungen als Werkstoff der Wahl für biodegradierbare Implantate, insbesondere Stents besonders vorteilhaft. Bei einem gegebenen Werkstoff ist die Duktilität abhängig von der Temperatur, der Beanspruchungsgeschwindigkeit, der Mehrachsigkeit des wirkenden mechanischen Spannungszustands und der Umgebung. Kennwerte der Duktilität sind z.B. die Bruchdehnung und -einschnürung, die Kerbschlagzähigkeit und die Bruchzähigkeit.

Bevorzugt weist die erfindungsgemäße Magnesiumlegierung eine Bruchdehnung von A5-Normproben bei Raumtemperatur von > 20% auf. Die Bruchdehnung (Reißdehnung, Formelzeichen ER oder A₅) ist die Bezeichnung für das prozentuale Verhältnis der Längenänderung ΔL (im Moment des Reißens) unter einer Zugbeanspruchung zur Ausgangslänge Lo.

Ein zweiter Aspekt der Erfindung bezieht sich auf ein Verfahren zur Herstellung einer feinkörnigen Magnesiumlegierung. Das Verfahren umfasst die Schritte:
(i) Bereitstellen eines Gemenges der Zusammensetzung

| | |
|---|---|
| Y: | 0,5 - 10 |
| Zn: | 0,5 - 6 |
| Ca: | 0,05 - 1 |
| Mn: | 0 - 0,5 |
| Ag: | 0 - 1 |
| Ce: | 0 - 1 |
| Zr: | 0 - 1 oder Si: 0 - 0,4 |

wobei sich die Angaben auf Gew.% am Gemenge beziehen und Mg sowie herstellungsbedingte Verunreinigungen den auf 100 Gew.% verbleibenden Restanteil am Gemenge einnehmen;
(ii) Urformen einer Magnesiumlegierung aus dem Gemenge durch Gießen;
und
(iii) Umformen der Magnesiumlegierung durch Pressen.

Mittels des erfindungsgemäßen Verfahrens ist es erstmalig möglich, sehr feinkörnige Magnesiumlegierungen im industriellen Maßstab herzustellen. Der Ansatz zeichnet sich schon dadurch aus, dass Rückgriff auf Erfahrungswerte aus an sich bekannten metallurgischen Verfahren genommen werden kann und so die Entwicklung eines industriellen Fertigungsprozesses wesentlich vereinfacht ist. Besonders bevorzugt erfolgt das Urformen im Schritt (ii) durch Stranggießen, da dieses Verfahren zu Werkstoffen hoher Homogenität führt. Ferner ist bevorzugt, wenn das Umformen im Schritt (iii) durch Strangpressen, insbesondere bei einer Temperatur im Bereich von 280°C - 420°C, besonders bevorzugt bei einer Temperatur im Bereich von 300°C - 400°C, erfolgt. Hinsichtlich der Gemengezusammensetzung ist gegebenenfalls an die gleichen, schon im Zusammenhang mit der Beschreibung der Magnesiumlegierung bevorzugten Zusammensetzungen anzupassen.

Gießen im Sinne der Erfindung bezieht sich auf ein Fertigungsverfahren, bei dem Werkstoffe in flüssigem oder breiigem Zustand in eine vorbereitete Hohlform (Gießform) gegossen werden, die das Negativ des abzugießenden Gussstücks bildet. Die Werkstoffe verfestigen sich in der Gießform und bilden diese als Positiv ab. Die Verfestigung erfolgt durch Erstarrung der metallischen Schmelze. Wenn druckloses Gießen zur Formfüllung nicht ausreicht, kann das Füllen unter Druck (Druckguss, Spritzguss) oder durch Nutzen der Fliehkraft (Schleuderguss) gegebenenfalls mit zusätzlicher Evakuierung der Gießform (Vakuumguss) erfolgen. Gießen im engeren Sinne der Erfindung bezieht sich auf Strangguss. Im Gegensatz zum Blockguss wird das Verfahren kontinuierlich durchgeführt. Dafür wird eine bodenlose gekühlte Kokille verwendet, in die das flüssige Metall gegossen wird. Innerhalb der Kokille erstarrt die Strangschale, die dann in Gießrichtung abgezogen wird und den flüssigen Kern umschließt. Nach dem Verlassen der Kokille wird die Strangschale weiter mit Wasser gekühlt, bis der Strang vollständig erstarrt ist.

Umformen (oder Verformen) ist die in der Metallbearbeitung gängige Bezeichnung für die plastische Deformation von metallischem Halbzeug unter dem Einfluss mechanischer Kräfte. Pressen bedeutet vorliegend das Zusammendrücken und Formen von Festkörpern, insbesondere die spanlose Umformung von Werkstoffen durch Druck in zumeist hydraulisch arbeitenden Maschinen. Die Bezeichnung Strangpressen bezieht sich auf ein Pressverfahren, bei dem ein Metallbolzen mit einem Stempel durch eine Matrize gedrückt wird. Dabei wird der Bolzen durch einen Aufnehmer umschlossen. Die äußere Form des Pressstrangs wird dabei durch die Matrize bestimmt. Durch das Einbringen verschieden geformter Dorne können auch Hohlräume erzeugt werden. Durch Strangpressen können Drähte, Rohre und Profile hergestellt werden.

Ein dritter Aspekt der Erfindung richtet sich auf eine nach dem zuvor beschriebenen Verfahren hergestellte Magnesiumlegierung.

Ein vierter Aspekt der Erfindung liegt in der Verwendung der nach dem Verfahren hergestellten Magnesiumlegierung oder einer auf andere Weise hergestellten feinkörnigen Magnesiumlegierung mit den obig genannten bevorzugten Gefügeeigenschaften in der Medizintechnik, insbesondere zur Herstellung eines biodegradierbaren Implantats. Letzteres ist vorzugsweise ein Stent.

### Ausführungsbeispiel 1

Ein Gemenge der Zusammensetzung (in Gew.%):

| | |
|---|---|
| Y: | 3,5 |
| Zn: | 0,85 |
| Ca: | 0,25 |
| Ag: | 0,5 |
| Mn: | 0,15 |
| Rest: | Mg und herstellungsbedingte Verunreinigungen |

wird bei 700°C in einem Tiegel unter Schutzatmosphäre geschmolzen und in eine zylindrische Werkzeugform mit einem Durchmesser von 25 mm gegossen. Das Gussstück mit einer Korngröße von etwa 150 Mikrometer wird anschließend auf 300° C erhitzt und durch eine Matrize auf einen Durchmesser von 5 mm extrudiert (Extrusionsverhältnis 25). Die dadurch erzielte Korngröße beträgt etwa 10 Mikrometer. Die vorliegenden intermetallischen Phasen sind unter anderem vom Typ Ca₂M₉₆Zn₃, Mg₃Y_{Z}Zn₃, Mg₃YZn₆ und AgMg₄ mit einer mittleren Größe von kleiner 3 Mikrometer und einer Gesamtmenge von weniger als 3 Volumenprozent. Die dadurch erzielte Duktilität gemessen in Bruchdehnungsprozent beträgt 24%, die Festigkeit (Zugfestigkeit) ergibt sich zu 270 MPa.

### Ausführungsbeispiel 2

Magnesiumlegierung der Zusammensetzung (in Gew.%):

| | |
|---|---|
| Y: | 6,5 |
| Zn: | 1,5 |
| Ca: | 0,25 |
| Ag: | 0,5 |
| Mn: | 0,15 |
| Zr: | 0,5 |
| Rest: | Mg und herstellungsbedingte Verunreinigungen |

wird bei 700°C in einem Tiegel unter Schutzatmosphäre geschmolzen und in eine zylindrische Werkzeugform mit einem Durchmesser von 25 mm gegossen. Das Gussstück mit einer Korngröße von etwa 50 Mikrometer wird anschließend auf 300°C erhitzt und durch eine Matrize auf einen Durchmesser von 5 mm extrudiert (Extrusionsverhältnis 25). Die dadurch erzielte Korngröße beträgt etwa 4 Mikrometer. Die vorliegenden intermetallischen Phasen sind vorwiegend vom Typ Ca₂Mg₆Zn₃, AgMg₄ und Zn₂Zr mit einer mittleren Größe von kleiner 1 Mikrometer und einer Gesamtmenge von weniger als 1 Volumenprozent. Die dadurch erzielte Duktilität gemessen in Bruchdehnungsprozent beträgt 26%, die Festigkeit (Streckgrenze) ergibt sich zu 190 MPa.

### Ausführungsbeispiel 3

Eine Magnesiumlegierung der Zusammensetzung (in Gew.%):

| | |
|---|---|
| Y: | 0,8 |
| Zn: | 2,0 |
| Ca: | 0,25 |
| Mn: | 0,15 |
| Rest: | Mg und herstellungsbedingte Verunreinigungen |

wurde bei 690° C in einem Magnesium-Ofen unter Schutzatmosphäre geschmolzen und in zylindrische Pressbolzen mit einem Durchmesser von 180 mm unter Wasserkühlung stranggegossen. Pressbolzen mit einer Korngröße von etwa 200 Mikrometer wurden anschließend auf 360° C erhitzt und durch eine Matrize auf einen Durchmesser von 20 mm extrudiert (Extrusionsverhältnis 30). Die dadurch erzielte Korngröße betrug etwa 7 Mikrometer. Die intermetallischen Phasen waren unter anderem vom Typ Ca₂Mg₆Zn₃, , Mg₃Y₂Zn₃ und Mg₃YZn₆ mit einer mittleren Größe von kleiner 3 Mikrometer und einer Gesamtmenge von weniger als 3 Volumenprozent. Die dadurch erzielte Duktilität gemessen in Bruchdehnungsprozent betrug 28%, die Festigkeit (Zugfestigkeit) ergab sich zu 260 MPa.

### Ausführungsbeispiel 4

Ein Gemenge der Zusammensetzung (in Gew.%):

| | |
|---|---|
| Y: | 2,0 |
| Zn: | 0,9 |
| Ca: | 0,25 |
| Mn: | 0,15 |
| Rest: | Mg und herstellungsbedingte Verunreinigungen |

wurde bei 690°C in einem Magnesium-Ofen unter Schutzatmosphäre geschmolzen und in zylindrische Pressbolzen mit einem Durchmesser von 180 mm unter Wasserkühlung stranggegossen (Verfahren gemäß erfindungsgemäße Lösung, zweiter Aspekt, (ii) Urformen). Pressbolzen mit einer Korngröße von etwa 250 Mikrometer wurden anschließend auf 380°C erhitzt und durch eine Matrize auf einen Durchmesser von 20 mm extrudiert (Extrusionsverhältnis 30, Verfahren gemäß erfindungsgemäße Lösung, zweiter Aspekt, (iii) Umformen). Die dadurch erzielte Korngröße betrug etwa 11 Mikrometer. Die vorliegenden intermetallischen Phasen sind unter anderem vom Typ Ca₂Mg₆Zn₃, Mg₃Y_{Z}Zn₃ und Mg₃YZn₆ mit einer mittleren Größe von kleiner 3 Mikrometer und einer Gesamtmenge von weniger als 3 Volumenprozent. Die dadurch erzielte Duktilität gemessen in Bruchdehnungsprozent betrug 28%, die Festigkeit (Zugfestigkeit) ergab sich zu 250 MPa.

### Ausführungsbeispiel 5

Ein Gemenge der Zusammensetzung (in Gew.%):

| | |
|---|---|
| Y: | 2,0 |
| Zn: | 0,9 |
| Ca: | 0,25 |
| Mn: | 0,15 |
| Zr: | 0,5 |
| Rest: | Mg und herstellungsbedingte Verunreinigungen |

wurde bei 690°C in einem Magnesium-Ofen unter Schutzatmosphäre geschmolzen und in zylindrische Pressbolzen mit einem Durchmesser von 180 mm unter Wasserkühlung stranggegossen (Verfahren gemäß erfindungsgemäße Lösung, zweiter Aspekt, (ii) Urformen). Pressbolzen mit einer Korngröße von etwa 120 Mikrometer wurden anschließend auf 400°C erhitzt und durch eine Matrize auf einen Durchmesser von 20 mm extrudiert (Extrusionsverhältnis 30, Verfahren gemäß erfindungsgemäße Lösung, zweiter Aspekt, (iii) Umformen). Die dadurch erzielte Korngröße betrug etwa 5 Mikrometer. Die vorliegenden intermetallischen Phasen sind unter anderem vom Typ Ca₂M9₆Zn₃, Mg₃Y₂Zn₃, Mg₃YZn₆, Zn₂Zr und Mn₂Zr mit einer mittleren Größe von kleiner 3 Mikrometer und einer Gesamtmenge von weniger als 3 Volumenprozent. Die dadurch erzielte Duktilität gemessen in Bruchdehnungsprozent betrug 22%, die Festigkeit (Zugfestigkeit) ergab sich zu 300 MPa.

## Patentansprüche

1. Magnesiumlegierung der Zusammensetzung
| | |
|---|---|
| Y: | 0.5 - 10, |
| Zn: | 0.5 - 6, |
| Ca: | 0.05 - 1, |
| Mn: | 0 - 0.5, |
| Ag: | 0 - 1, |
| Ce: | 0 - 1, |
| Zr: | 0 - 1 oder Si: 0 - 0.4 |
wobei sich die Angaben auf Gew.% an der Legierung beziehen und Mg sowie herstellungsbedingte Verunreinigungen den auf 100 Gew.% verbleibenden Restanteil an der Legierung einnehmen.

2. Magnesiumlegierung nach Anspruch 1, bei der die Magnesiumlegierung ein oder mehrere intermetallische Phasen bestehend aus
(i) Mg und einem oder mehreren Elementen ausgewählt aus der Gruppe: Zn, Ca, Mn, Ag, Ce, Zr, Si und Y; oder
(ii) 2 oder mehr Elementen ausgewählt aus der Gruppe: Zn, Ca, Mn, Ag, Ce, Zr, Si und Y
enthält.

3. Magnesiumlegierung nach Anspruch 2, enthaltend ein oder mehrere intermetallische Phasen ausgewählt aus der Gruppe:
Ca₂Mg₆Zn₃, AgMg₄, Mn₂Zr, ZnZr, Zn₂Zr, MgZn, MgZn₂, Mg₂Si, Mg₃Y₂Zn₃, Mg₃YZn₆, Mg₁₂YZn und Mg₂₄Y₅.

4. Magnesiumlegierung nach Anspruch 2, bei der ein Volumenanteil der intermetallischen Phasen an der Magnesiumlegierung < 3 Vol.% ist.

5. Magnesiumlegierung nach Anspruch 4, bei der der Volumenanteil der intermetallischen Phasen an der Magnesiumlegierung < 2 Vol.% ist.

6. Magnesiumlegierung nach Anspruch 2, bei der eine Korngröße der intermetallischen Phasen < 3 µm ist.

7. Magnesiumlegierung nach Anspruch 2, bei der eine Korngröße der Legierung < 20 µm ist.

8. Magnesiumlegierung nach Anspruch 7, bei der die Korngröße der Legierung < 10 µm ist.

9. Magnesiumlegierung nach einem der vorhergehenden Ansprüche, bei der
| | |
|---|---|
| Y: | 0,5 - 4 |
ist.

10. Magnesiumlegierung nach einem der vorhergehenden Ansprüche, bei der
| | |
|---|---|
| Zn: | 0,5 - 3,0 |
ist.

11. Magnesiumlegierung nach einem der vorhergehenden Ansprüche, bei der
| | |
|---|---|
| Ca: | 0,05 - 0,3 |
ist.

12. Magnesiumlegierung nach einem der vorhergehenden Ansprüche, bei der
| | |
|---|---|
| Mn | 0 - 0,25 |
ist.

13. Magnesiumlegierung nach einem der vorhergehenden Ansprüche, bei der
| | |
|---|---|
| Ag: | 0,05 - 0,6 |
ist.

14. Magnesiumlegierung nach einem der vorhergehenden Ansprüche, bei der
| | |
|---|---|
| Ce: | 0 - 0,5 |
ist.

15. Magnesiumlegierung nach einem der vorhergehenden Ansprüche, bei der
| | |
|---|---|
| Zr: | 0 - 0,7 |
ist.

16. Magnesiumlegierung nach einem der Ansprüche 1 - 14, bei der
| | |
|---|---|
| Si: | 0 - 0,25 |
ist.

17. Verfahren zur Herstellung einer feinkörnigen Magnesiumlegierung, umfassend die Schritte:
(i) Bereitstellen eines Gemenges der Zusammensetzung
| | |
|---|---|
| Y: | 0,5 - 10 |
| Zn: | 0,5 - 6 |
| Ca: | 0,05 - 1 |
| Mn: | 0 - 0,5 |
| Ag: | 0 - 1 |
| Ce: | 0 - 1 |
| Zr: | 0 - 1 oder Si: 0 - 0,4 |
wobei sich die Angaben auf Gew.% am Gemenge beziehen und Mg sowie herstellungsbedingte Verunreinigungen den auf 100 Gew.% verbleibenden Restanteil am Gemenge einnehmen;
(ii) Urformen einer Magnesiumlegierung aus dem Gemenge durch Gießen; und
(iii) Umformen der Magnesiumlegierung durch Pressen.

18. Verfahren nach Anspruch 17, bei dem das Urformen im Schritt (ii) durch Stranggießen erfolgt.

19. Verfahren nach Anspruch 17, bei dem das Umformen im Schritt (iii) durch Strangpressen erfolgt.

20. Verfahren nach Anspruch 19, bei dem das Strangpressen bei einer Temperatur im Bereich von 280°C - 420°C erfolgt.

21. Verfahren nach Anspruch 20, bei dem das Strangpressen bei einer Temperatur im Bereich von 300°C - 400°C erfolgt.

22. Verwendung einer nach dem Verfahren gemäß einem der Ansprüche 17 - 21 hergestellten Magnesiumlegierung oder einer Magnesiumlegierung nach Anspruch 1 - 16 zur Herstellung eines biodegradierbaren Implantats.

## Claims

1. A magnesium alloy, having the following composition:
| | |
|---|---|
| Y: | 0.5 - 10, |
| Zn: | 0.5 - 6, |
| Ca: | 0.05 - 1, |
| Mn: | 0 - 0.5, |
| Ag: | 0 - 1, |
| Ce: | 0 - 1, |
| Zr: | 0 - 1 or Si: 0 - 0.4, |
wherein the amounts are based on weight percent in the alloy, and Mg and manufacturing-related impurities account for the remaining content in the alloy that is missing to make up 100% by weight.

2. The magnesium alloy according to claim 1, wherein the magnesium alloy includes one or more intermetallic phases comprising:
(i) Mg and one or more elements selected from the group consisting of: Zn, Ca, Mn, Ag, Ce, Zr, Si and Y; or
(ii) two or more elements selected from the group consisting of: Zn, Ca, Mn, Ag, Ce, Zr, Si and Y.

3. The magnesium alloy according to claim 2, comprising one or more intermetallic phases selected from the group consisting of:
Ca₂Mg₆Zn₃, AgMg₄, Mn₂Zr, ZnZr, Zn₂Zr, MgZn, MgZn₂, Mg₂Si, Mg₃Y₂Zn₃, Mg₃YZn₆, Mg₁₂YZn and Mg₂₄Y₅.

4. The magnesium alloy according to claim 2, wherein a volume fraction of the intermetallic phases in the magnesium alloy is < 3% by volume.

5. The magnesium alloy according to claim 4, wherein the volume fraction of the intermetallic phases in the magnesium alloy is < 2% by volume.

6. The magnesium alloy according to claim 2, wherein a particle size of the intermetallic phases is < 3 µm.

7. The magnesium alloy according to claim 2, wherein a particle size of the alloy is < 20 µm.

8. The magnesium alloy according to claim 7, wherein the particle size of the alloy is < 10 µm

9. A magnesium alloy according to any one of the preceding claims, wherein:
Y is: 0.5 - 4.

10. A magnesium alloy according to any one of the preceding claims, wherein:
Zn is: 0.5 - 3.0.

11. A magnesium alloy according to any one of the preceding claims, wherein:
Ca is: 0.05 - 0.3.

12. A magnesium alloy according to any one of the preceding claims, wherein:
Mn is: 0 - 0.25.

13. A magnesium alloy according to any one of the preceding claims, wherein:
Ag is: 0.05 - 0.6.

14. A magnesium alloy according to any one of the preceding claims, wherein:
Ceis: 0 - 0.5.

15. A magnesium alloy according to any one of the preceding claims, wherein:
Zr is: 0-0.7.

16. A magnesium alloy according to any one of claims 1 to 14, wherein:
Si is: 0 - 0.25.

17. A method for producing a fine-grained magnesium alloy, comprising the following steps:
(i) providing a mixture having the following composition:
| | |
|---|---|
| Y: | 0.5 - 10, |
| Zn: | 0.5 - 6, |
| Ca: | 0.05 - 1, |
| Mn: | 0 - 0.5, |
| Ag: | 0 - 1, |
| Ce: | 0 - 1, |
| Zr: | 0 - 1 or Si: 0 - 0.4, |
wherein the amounts are based on weight percent in the mixture, and Mg and manufacturing-related impurities account for the remaining content in the mixture that is missing to make up 100% by weight;
(ii) primary shaping a magnesium alloy from the mixture by way of casting; and
(iii) reshaping the magnesium alloy by way of pressing.

18. The method according to claim 17, wherein the primary shaping in step (ii) is carried out by way of continuous casting.

19. The method according to claim 17, wherein the reshaping in step (iii) is carried out by way of extrusion molding.

20. The method according to claim 19, wherein the extrusion molding is carried out at a temperature in the range of 280° to 420°C.

21. The method according to claim 20, wherein the extrusion molding is carried out at a temperature in the range of 300° to 400°C.

22. Use of a magnesium alloy produced by the method according to any one of claims 17 to 21, or of a magnesium alloy according to claims 1 to 16, for producing a biodegradable implant.

## Revendications

1. Alliage de magnésium avec la composition suivante :
| | |
|---|---|
| Y: | 0,5 - 10 |
| Zn: | 0,5 - 6 |
| Ca : | 0,05 - 1 |
| Mn: | 0 - 0,5 |
| Ag: | 0 - 1 |
| Ce: | 0 - 1 |
| Zr: | 0 - 1 ou Si: 0 - 0,4 |
dans lequel les indications sont fournies en % en poids de l'alliage, et Mg ainsi que les impuretés dues à la fabrication constituent la part résiduelle de l'alliage pour aller à 100% en poids.

2. Alliage de magnésium selon la revendication 1, dans lequel l'alliage de magnésium contient une ou plusieurs phases intermétalliques, constituées de
(i) Mg ou l'un ou plusieurs des éléments sélectionnés parmi le groupe suivant : Zn, Ca, Mn, Ag, Ce, Zr, Si et Y; ou
(ii) 2 ou plusieurs éléments sélectionnés parmi le groupe : Zn, Ca, Mn, Ag, Ce, Zr, Si et Y.

3. Alliage de magnésium selon la revendication 2, contenant une ou plusieurs phases intermétalliques sélectionnées parmi le groupe suivant:
Ca₂Mg₆Zn₃, AgMg₄, Mn₂Zr, ZnZr, Zn₂Zr, MgZn, MgZn₂, Mg₂Si, Mg₃Y₂Zn₃, Mg₃YZn₆, Mg₁₂YZn et Mg₂₄Y₅.

4. Alliage de magnésium selon la revendication 2, dans lequel la fraction volumique des phases intermétalliques dans l'alliage de magnésium est < 3% en volume.

5. Alliage de magnésium selon la revendication 4, dans lequel la fraction volumique des phases intermétalliques dans l'alliage de magnésium est < 2% en volume.

6. Alliage de magnésium selon la revendication 2, dans lequel une taille de grain des phases intermétalliques est < 3 µm.

7. Alliage de magnésium selon la revendication 2, dans lequel une taille de grain de l'alliage est < 20 µm.

8. Alliage de magnésium selon la revendication 7, dans lequel la taille de grain de l'alliage est < 10 µm.

9. Alliage de magnésium selon l'une des revendications précédentes, dans lequel
| | |
|---|---|
| Y: | 0,5 - 4 |

10. Alliage de magnésium selon l'une des revendications précédentes, dans lequel
| | |
|---|---|
| Zn: | 0,5 - 3,0 |

11. Alliage de magnésium selon l'une des revendications précédentes, dans lequel
| | |
|---|---|
| Ca: | 0,05 - 0,3 |

12. Alliage de magnésium selon l'une des revendications précédentes, dans lequel
| | |
|---|---|
| Mn: | 0 - 0,25 |

13. Alliage de magnésium selon l'une des revendications précédentes, dans lequel
| | |
|---|---|
| Ag: | 0,05 - 0,6 |

14. Alliage de magnésium selon l'une des revendications précédentes, dans lequel
| | |
|---|---|
| Ce: | 0 - 0,5 |

15. Alliage de magnésium selon l'une des revendications précédentes, dans lequel
| | |
|---|---|
| Zr: | 0 - 0,7 |

16. Alliage de magnésium selon l'une des revendications 1 - 14, dans lequel
| | |
|---|---|
| Si: | 0 - 0,25 |

17. Procédé pour la fabrication d'un alliage de magnésium à grain fin, comprenant les étapes suivantes :
(i) Mise à disposition d'un mélange avec la composition suivante
| | |
|---|---|
| Y: | 0,5 - 10 |
| Zn: | 0,5 - 6 |
| Ca: | 0,05 - 1 |
| Mn: | 0 - 0,5 |
| Ag: | 0 - 1 |
| Ce: | 0 - 1 |
| Zr: | 0 - 1 ou Si : 0 - 0,4 |
dans lequel les indications sont fournies en % en poids du mélange, et Mg ainsi que les impuretés dues à la fabrication constituent la part résiduelle de l'alliage pour aller à 100% en poids ;
(ii) Formage d'un alliage de magnésium par coulée à partir du mélange ; et
(iii) Formage de l'alliage de magnésium par pressage.

18. Procédé selon la revendication 17, dans lequel le formage dans l'étape (ii) est effectué par coulée continue.

19. Procédé selon la revendication 17, dans lequel le formage dans l'étape (iii) est effectué par extrusion.

20. Procédé selon la revendication 19, dans lequel l'extrusion est effectuée à une température comprise entre 280°C et 420°C.

21. Procédé selon la revendication 20, dans lequel l'extrusion est effectuée à une température comprise entre 300°C et 400°C.

22. Utilisation d'un alliage de magnésium fabriqué à l'aide du procédé selon l'une des revendications 17 à 21, ou d'un alliage de magnésium selon l'une des revendications 1 à 16 pour la fabrication d'un implant biodégradable.
